(19) Europäisches Patentamt
European Patent Office
Office européen des brevets

(11) Veröffentlichungsnummer : **0 363 766 B1**

(12) # EUROPÄISCHE PATENTSCHRIFT

(45) Veröffentlichungstag der Patentschrift : **10.04.91 Patentblatt 91/15**

(51) Int. Cl.$^5$ : **C07D 249/08,** C07D 233/56, A61K 31/41, A61K 31/415

(21) Anmeldenummer : **89118155.4**

(22) Anmeldetag : **30.09.89**

(54) **Azolylmethylcyclopropane und ihre Verwendung als Pflanzenschutzmittel.**

Die Akte enthält technische Angaben, die nach dem Eingang der Anmeldung eingereicht wurden und die nicht in dieser Patentschrift enthalten sind.

(30) Priorität : **10.10.88 DE 3834437**

(43) Veröffentlichungstag der Anmeldung : **18.04.90 Patentblatt 90/16**

(45) Bekanntmachung des Hinweises auf die Patenterteilung : **10.04.91 Patentblatt 91/15**

(84) Benannte Vertragsstaaten : **AT BE CH DE ES FR GB GR IT LI NL SE**

(56) Entgegenhaltungen :
**EP-A- 0 095 285**
**DE-A- 3 307 477**
**DE-A- 3 307 479**

(73) Patentinhaber : **BASF Aktiengesellschaft**
**Carl-Bosch-Strasse 38**
**W-6700 Ludwigshafen (DE)**

(72) Erfinder : **Seele, Rainer, Dr.**
**Leiblstrasse 3**
**W-6701 Fussgoenheim (DE)**
Erfinder : **Kober, Reiner, Dr.**
**Im Schlittweg 20**
**W-6701 Fussgoenheim (DE)**
Erfinder : **Karbach, Stefan, Dr.**
**Grundwiesenweg 44**
**W-6730 Neustadt (DE)**
Erfinder : **Sauter, Hubert, Dr.**
**Neckarpromenade 20**
**W-6800 Mannheim 1 (DE)**
Erfinder : **Ammermann, Eberhard, Dr.**
**Sachsenstrasse 3**
**W-6700 Ludwigshafen (DE)**
Erfinder : **Lorenz, Gisela, Dr.**
**Erlenweg 13**
**W-6730 Neustadt (DE)**
Erfinder : **Rademacher, Wilhelm, Dr.**
**Ausstrasse 1**
**W-6703 Limburgerhof (DE)**
Erfinder : **Jung, Johann, Prof. Dr.**
**Hardenburgstrasse 19**
**W-6703 Limburgerhof (DE)**

**EP 0 363 766 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Azolverbindungen, Verfahren zu ihrer Herstellung und diese enthaltende Fungizide und wachstumsregulierende Mittel.

Es ist bekannt, 1-(1,2,4-Triazol-1-ylmethyl)-1-(4-fluorphenyl)-2-(2,4-dichlorphenyl)-cyclopropan oder 1-(1,2,4-Triazol-1-ylmethyl)-1-(4-chlorphenyl)-2-(2-chlorphenyl)-cyclopropan (EP 121 081) als Fungizide zu verwenden. Die fungiziden Wirkungen sind jedoch unbefriedigend.

Es wurde nun gefunden, daß Verbindungen der Formel I

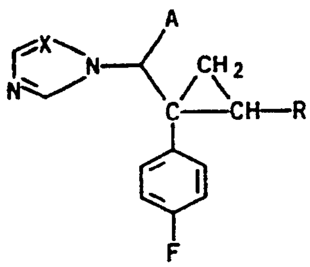

I,

in welcher

R $C_1$-$C_8$-Alkyl, $C_3$-$C_8$-Cycloalkyl, $C_5$-$C_8$-Cycloalkenyl, Tetrahydropyranyl, Norbornyl, Pyridyl, Naphthyl, Biphenyl oder Phenyl bedeutet, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,

A den Rest H, F, Cl oder Br bedeutet,

x den Rest CH oder N bedeutet,

sowie deren für Pflanzen verträgliche Säureadditionssalze oder Metallkomplexe außer den Verbindungen, in denen gleichzeitig A Wasserstoff und R 2,4-Dichlorphenyl bedeuten eine bessere fungizide Wirkung besitzen als bekannte Azolverbindungen und eine gute wachstumsregulierende Wirkung haben.

Die Verbindungen der Formel I enthalten chirale Zentren und werden im allgemeinen in Form von Diastereomerengemischen erhalten. Die Diastereomeren lassen sich bei den neuen Verbindungen in üblicher Weise, beispielsweise aufgrund ihrer unterschiedlichen Löslichkeit oder durch Säulenchromatographie trennen und in reiner Form isolieren. Aus einem solchen isolierten Diastereomeren kann man mit bekannten Methoden einheitliche Enantiomere erhalten. Als Wirkstoffe kann man sowohl die einheitlichen Diastereomere bzw. Enantiomere wie auch deren bei der Synthese anfallenden Gemische verwenden.

Alle diese Verbindungen und ihre Gemische werden von der vorliegenden Erfindung umfaßt.

R bedeutet beispielsweise Methyl, Ethyl, Isopropyl, n-Propyl, n-Butyl, sec.-Butyl, tert.-Butyl, n-Pentyl, Neopentyl, Hexyl, Octyl, Trifluormethyl, Trichlormethyl, Phenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromhenyl, 2,3-Dichlorphenyl, 2,5-Dichlorphenyl, 2,6-Dichlorphenyl, 2-Chlor-6-fluorphenyl, 2-Methoxyphenyl, 3-Methoxyphenyl, 4-Methoxyphenyl, 2,4-Dimethoxyphenyl, 4-Ethylphenyl, 4-Isopropylphenyl, 4-tert.-Butylphenyl, 4-tert.-Butyloxyphenyl, 2-Chlor-4-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Trifluormethylphenyl, 3-Trifluoromethylphenyl, 4-Trifluoromethylphenyl, Cyclopropyl, Cyclobutyl, Cyclopentyl, Cyclohexyl, Tetrahydropyranyl, 2-Cyclohexenyl, 3-Cyclohexenyl, Norbornyl.

Säureadditionssalze sind beispielsweise die Hydrochloride, Bromide, Sulfate, Nitrate, Phosphate, Oxalate, oder Dodecylbenzolsulfonate. Die Wirksamkeit der Salze geht auf das Kation zurück, so daß es auf das Anion i.a. nicht ankommt. Die erfindungsgemäßen Wirkstoffsalze werden hergestellt durch Umsetzung der Azolymethyloxirane (I) mit dem Säuren.

Metallkomplexe der Wirkstoffe I oder ihre Salze können z.B. mit Kupfer, Zink, Zinn, Mangan, Eisen, Kobalt oder Nickel gebildet werden, indem man die Azolylmethylcyclopropane mit entsprechenden Metallsalzen umsetzt, z.B. mit Kupfersulfat, Zinkchlorid, Zinnchlorid, Mangansulfat.

Die Verbindungen der Formel I, in denen A den Rest H bedeutet, können z.B. hergestellt werden, indem man

a) eine Verbindung der Formel II

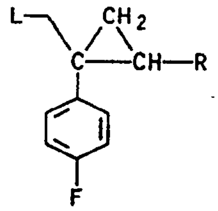

II,

in welcher R die angegebene Bedeutung hat und L eine nukleophil substituierbare Abgansgruppe (z.B. Halogen, OH) bedeutet, mit einer Verbindung der Formel III

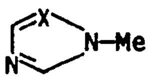

III,

in der Me ein Wasserstoffatom, ein Metallatom (z.B. Na, K) oder eine Trimethylsilylgruppe bedeutet und x die oben angegebene Bedeutung hat, zur Umsetzung bringt, oder
b) eine Verbindung der Formel IV

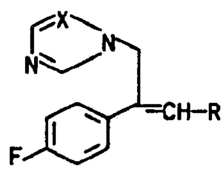

IV,

in welcher R und x die oben angegebenen Bedeutungen haben, in das Cyclopropan überführt.

Die Reaktion a) erfolgt – falls Me ein Wasserstoffatom bedeutet – z.B. gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120°C. Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon, ferner Dimethylsulfoxid, Sulfolan oder entsprechende Gemische.

Geeignete Basen, die gegebenenfalls auch als säurebindende Mittel bei der Reaktion verwendet werden können, sind beispielsweise Alkalihydroxid wie Lithium-, Natrium- oder Kaliumhydroxid, Alkalicarbonate wie Natrium-, Kalium- oder Cäsiumcarbonat oder Natrium-, Kalium- oder Cäsiumhydrogencarbonat, Pyridin oder 4-Dimethylaminopyridin. Es können aber auch andere übliche Basen verwendet werden.

Als Reaktionsbeschleuniger kommen vorzugsweise Metallhalogenide wie Natriumjodid oder Kaliumjodid, quaternäre Ammoniumsalze wie Tetrabutylammoniumchlorid, -bromid, -jodid oder -hydrogensulfat, Benzyltriethylammoniumchlorid oder -bromid oder Kronenether wie 12-Krone-4, 15-Krone-5, 18-Krone-6, Dibenzo-18-krone-6 oder Dicyclohexano-18-Krone-6 in Frage.

Die Umsetzung wird im allgemeinen bei Temperaturen zwischen 10 und 120°C, drucklos oder unter Druck, kontinuierlich oder diskontinuierlich durchgeführt.

Ist Me ein Metallatom, wird die Reaktion a) gegebenenfalls in Gegenwart eines Lösungs- oder Verdünnungsmittels und gegebenenfalls unter Zusatz einer starken anorganischen oder organischen Base bei Temperaturen zwischen −10 und 120°C durchgeführt. Zu den bevorzugten Lösungs- oder Verdünnungsmitteln gehören Amide wie Dimethylformamid, Diethylformamid, Dimethylacetamid, Diethylacetamid, N-Methylpyrrolidon, Hexamethylphosphortriamid, Sulfoxide wie Dimethylsulfoxid und schließlich Sulfolan.

Geeignete Basen, die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium- oder Kalium-tert.-butoxide, Lithium-, Natrium- oder Kalium-triphenylmethyl und Naphthalinlithium, -natrium oder -kalium.

Für die Reaktion b) kommen als Verdünnungsmittel polare organische Lösungsmittel wie Nitrile, z.B. Acetonitril, Sulfoxide, z.B. Dimethylsulfoxid, Formamide, z.B. Dimethylformamid, ketone, z.B. Aceton, Ether, z.B. Diethylether, Tetrahydrofuran und insbesondere Chlorkohlenwasserstoffe, z.B. Methylenchlorid und Chloroform, in Frage.

Die Reaktion b) erfolgt z.B. unter Verwendung eines Trimethylsulfoxoniumsalzes z.B. des Trimethylsulfoxoniumjodids und von Alkali z.B. einem Alkalialkoholat, z.B. Kalium-tert.-butanolat.

Man arbeitet im allgemeinen zwischen 0 und 100°C, vorzugsweise bei 20 bis 80°C. Bei Anwesenheit eines Lösungsmittels wird zweckmäßig beim Siedepunkt des jeweiligen Lösungsmittels gearbeitet.

Die Verbindungen der Formel II, in denen L eine nukleophil substituierbare Abgangsgruppe darstellt, lassen sich nach bekannten Synthesemethoden aus den Verbindungen der Formel II (L = OH) durch Umsetzung mit Halogenüberträgern wie Chlorwasserstoff, Bromwasserstoff, Thionylchlorid, Thionylbromid, Acetylbromid, Phosphortribromid oder dem Triphenylphosphin-Brom-Komplex oder Sulfonsäurechloriden wie Methan-, Trifluormethan-, 2,2,2-Trifluorethan-, Nonafluorbutan-, 4-Methylbenzol-, 4-Brombenzol-, 4-Nitrobenzol- oder Benzolsulfonsäurechlorid, gegebenenfalls in Gegenwart eines inerten Lösungsmittels und gegebenenfalls in Anwesenheit einer organischen oder anorganischen Base, wobei diese auch gleichzeitig Lösungsmittel sein kann, herstellen (vgl. z.B. Houben-Weyl-Müller, Methoden oder organischen Chemie, Bd 5/3 Stuttgart 1964, S. 760 ff. ; Bd. 5/4, Stuttgart 1960, S. 354 ff. ; Bd. 9, Stuttgart 1955, S. 388, 633, J. org. Chem. 35, 3195 (1970).

Hydroxycyclopropane der Formel II (L = OH) erhält man nach folgendem Schema :

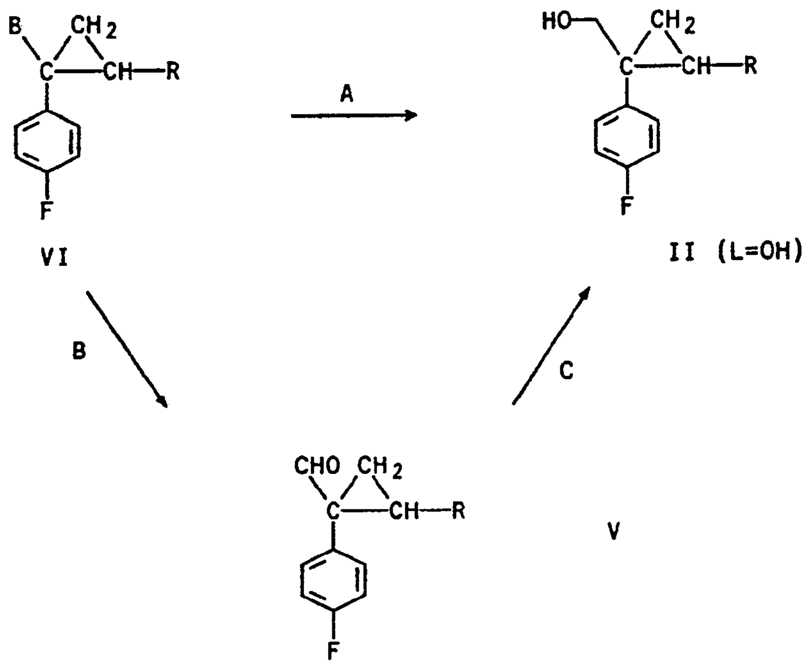


VI

II (L=OH)

V

B = CN, COOH, eine Estergruppe oder eine acetalisierte Aldehydgruppe Nitrile der Formel VI (B = CN) lassen sich sauer oder alkalisch bei Temperaturen zwischen 20 und 120°C in Gegenwart eines Lösungsmittels zu den entsprechenden Carbonsäurederivaten VI (B = COOH) solvolysieren, die durch Reduktion mit einem komplexen Hydrid, z.B. Lithiumaluminiumhydrid oder Natriumborhydrid – im Falle eines Anhydrids – oder mit Wasserstoff in Gegenwart eines Katalysators drucklos oder unter Druck nach allgemein bekannten Methoden in die Alkohole II (L = OH) überführt werden (Verfahren A).

Alternativ kann aus dem Nitril VI (B = CN) durch Reduktion mit Diisobutylaluminiumhydrid (vgl. E. Winterfeldt, Synthesis 1975, 617) der Aldehyd V hergestellt werden (Verfahren B), der mit einem komplexen Hydrid, z.B. Natriumborhydrid oder Lithiumaluminiumhydrid oder mit Wasserstoff in Gegenwart eines Katalysators weiterreduziert wird zum Alkohol der Formel II (L = OH) (Verfahren C).

Cyclopropylderivate der Formel VI, in der R die oben angegebene Bedeutung hat und B eine Nitrilgruppe, Estergruppe oder eine gegebenenfalls substituierte acetalisierte Aldehydgruppe darstellt, lassen sich bevorzugt durch Cyclopropanierung α,β-ungesättigter Nitrile, Carbonsäurederivate oder Aldehydderivate der Formel VII

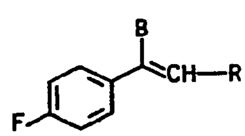

VII

in welcher B und R die oben angegebene Bedeutung haben, herstellen. Für diese Reaktion eignen sich besonders Trimethylsulfoxoniumsalze, die in einem inerten Lösungsmittel in Gegenwart einer starken Base, z.B. eines Alkalialkoholates wie Kalium-tert.-butanolat, mit einer Verbindung der Formel VII, in welcher B und R oben angegebene Bedeutung haben, ungesetzt werden (vgl. Corey, Chaykovsky, J. Am, Chem. Soc. 1962, 64, 3782).

Weitere Verfahren zur Herstellung von substituierten Cyclopropanen durch intramolekulare Reaktion oder durch Umsetzung von Olefinen mit Methylengruppenübertragungsreagenzien wie Diazomethan oder nach Simmon-Smith sind bekannt (vgl. z.B. D. Wendisch in Houben-Weyl-Müller, Methoden der organischen Chemie, Stuttgart 1971, Bd 4/3, S. 32-148).

Die Verbindungen der Formel I, in denen A den Rest F, Cl oder Br bedeutet, können z.B. hergestellt werden, indem man eine Verbindung der Formel V

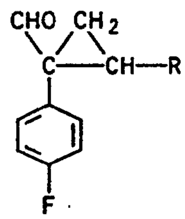

V

in welcher R die oben angegebene Bedeutung hat, mit einer Verbindung der Formel III

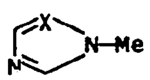

III,

in der X und Me die oben angegebene Bedeutung haben, in Gegenwart des entsprechend Thionylhalogenids zur Umsetzung bringt. Z.B. für die Herstellung von Verbindungen mit A = Cl verwendet man Thonylchlorid, für A = Br verwendet man Thionylbromid.

Die Umsetzung erfolgt gegebenenfalls in Gegenwart eines Lösungs- oder Verdünnungsmittels bei Temperaturen zwischen –30 und 80°C. Zu den bevorzugten Lösungs- und Verdünnungsmitteln gehören Nitrile wie Acetonitril oder Propionitril, Ether wie Tetrahydrofuran, Diethylether, Dimethoxyethan, Dioxan oder Diisopropylether und insbesondere Kohlenwasserstoffe und Chlorkohlenwasserstoffe wie Pentan, Hexan, Toluol, Methylenchlorid, Chloroform, Tetrachlorkohlenstoff, Dichlorethan oder entsprechende Gemische.

Die Verbindungen in Formel VII lassen sich entsprechend allgemein bekannter Verfahren zur Olefinsynthese (Houben-Weyl-Müller, Methoden der organischen Chemie, Georg Thieme Verlag, Stuttgart 1972, Bd V, 1b) herstellen.

Beispiele

Die folgenden Beispiele erläutern die Herstellung der Wirkstoffe.

I Herstellung der Ausgangsstoffe

Beispiel A

Zu einer Lösung von 50 g 4-Fluorphenylacetonitril in 500 ml Ethanol wird unter Stickstoffatmosphäre 52 g 2-Chlorbenzaldehyd gegeben. Das Reaktionsgemisch wird auf 10°C gekühlt und schnell 5 g Natriummethanolat zugegeben, wobei die Temperatur in der Lösung 50°C nicht übersteigt. Nach einstündigem Rühren bei Raumtemperatur (20°C) wird der entstandene Niederschlag abgesaugt, mit n-Hexan sowie Ethanol gewaschen und anschließend getrocknet. Man erhält 91,7 g (96%) E-1-(4-Fluorphenyl)-2-(2-chlorphenyl)-acrylsäurenitril.

Beispiel B

Zu einer Lösung von 91,7 g E-1-(4-Fluorphenyl)-2-(2-chlorphenyl)-acrylsäurenitril in 100 ml Methylenchlorid werden 73,5 g Trimethylsulfoniummethylsulfat und 150 ml Natronlauge (50%ig) gegeben. Nachdem das Reaktionsgemisch 48 Stunden bei Raumtemperatur gehalten wurde, wird der Lösung 200 ml Wasser zugesetzt

und die organische Phase abgetrennt. Die verbleibende wäßrige Phase wird mit Methylenchlorid extrahiert und die gesammelten organischen Phasen mit Wasser gewaschen. Nach Trocknen über Natriumsulfat und Einengen der organischen Phase werden 87,4 g (90%) cis-1-(4-Fluorphenyl)-2-(2-chlorphenyl)-cyclopropylcyanid mit dem Schmelzpunkt 108-115°C erhalten.

Beispiel C

32,1 g cis-1-(4-Fluorphenyl)-2-(2-chlorphenyl)-cyclopropylcyanid werden in 250 ml Toluol aufgenommen und unter Stickstoffatmosphäre bei 0 bis 4°C tropfenweise mit 100 ml Diisobutylaluminiumhydrid (1,2 molar in Toluol) versetzt. Nach einstündigem Rühren bei Raumtemperatur wird der Reaktionslösung schnell 500 ml Schwefelsäure (5%ig) zugesetzt und mehrmals mit Methyl-tert.-butylether extrahiert. Die organische Phase wird zweimal mit Wasser gewaschen, über Natriumsulfat getrocknet und eingeengt. Man erhält 28,2 g (89%) cis-1-(4-Fluorphenyl)-2-(2-chlorphenyl)-cyclopropylcarbaldehyd mit dem Schmelzpunkt 79-80°C.

Beispiel D

26,2 g cis-1-(4-Fluorphenyl)-2-(2-chlorphenyl)-2-cyclopropyl-carbaldehyd wird in 100 ml Ethanol gelöst und bei Raumtemperatur 1,22 g Natriumborhydrid zugesetzt. Danach wird das Lösungsmittel im Vakuum abgedampft, der Rückstand in Methylenchlorid aufgenommen, mit Wasser gewaschen, über Natriumsulfat getrocknet und die Lösung eingedampft. Umkristallisation des Rückstandes aus Toluol liefert 26,4 g (98%) cis-1-Hydroxymethyl-1-(4-fluor-phenyl)-2-(2-chlorphenyl)-cyclopropan mit dem Schmelzpunkt 86-89°C.

Beispiel E

Zu einer Lösung von 26,4 g cis-1-Hydroxymethyl-1-(4-fluor-phenyl)-2-(2-chlorphenyl)-cyclopropan in 150 ml Methylenchlorid und 12,6 g Triethylamin werden bei Raumtemperatur 22,7 g 4-Methylbenzolsulfonsäurechlorid gegeben. Nach 24 Stunden wird das Reaktionsgemisch mit wäßriger Natriumhydrogencarbonat-Lösung und Wasser gewaschen, über Natriumsulfat getrocknet und im Vakuum eingedampft. Aus dem Rückstand erhielt man 40,2 g (98%) cis-1-(4-Methylphenylsulfonyloxymethyl)-1-(4-fluorphenyl)-2-(2-chlorphenyl)-cyclopropan, das anschließend mit Triazol weiter verarbeitet wurde.

II Herstellung der Endprodukte

Beispiel 1

Eine Lösung von 6,9 g 1,2,4-Triazol in 100 ml N-Methylpyrrolidon wird mit 4,8 g Natriumhydroxid versetzt und für 30 Minuten auf 50°C erwärmt. Nachdem das Reaktionsgemisch auf Raumtemperatur gekühlt wurde, wird der Lösung 21,5 g cis-1-(4-Methylphenyl-sulfonyloxymethyl)-1-(4-fluorphenyl)-2-(2-chlorphenyl)-cyclopropan, das in 50 ml N-Methylpyrrolidon gelöst ist, langsam zugetropft und 12 Stunden bei Raumtemperatur gerührt. Anschließend wird 200 ml Wasser zugegeben und mehrmals mit Methyl-tert.-butylether extrahiert, die organische Phase zweimal mit Wasser gewaschen, über Natriumsulfat getrocknet und eingeengt. Der verbleibende Rückstand wird einer Chromatographie an Kieselgel mit n-Hexan/Essigester = 9 : 1 unterworfen, wobei 12 g (76%) cis-1-(1,2,4-Triazol-1-yl-methyl)-1-(4-fluorphenyl)-2-(2-chlorphenyl)-cyclopropan, Fp.: 97-101°C erhalten werden (Verbindung Nr. 1).

Beispiel 2

Zu einer Lösung von 15,8 g 1,2,4-Triazol in 100 ml Methylenchlorid wird bei 0°C unter Stickstoffatomosphäre 6,8 g Trionylchlorid zugesetzt. Nach beendeter Zugabe wird bei Raumtemperatur für 30 Minuten gerührt und anschließend 10 g cis-1-(4-Fluorphenyl)-2-(2-fluorphenyl)-cyclopropylcarbaldehyd zugegeben. Nachdem das Reaktionsgemisch 12 bis 15 Stunden bei Raumtemperatur rührte, wird der Lösung 100 ml Wasser zugesetzt und die organische Phase abgetrennt. Die verbleibende wäßrige Phase wird zweimal mit Methylenchlorid ausgeschüttelt und die gesammelten organischen Phasen zweimal mit gesättigter Natriumhydrogencarbonat-Lösung gewaschen. Die isolierte Phase wird daraufhin über Natriumsulfat getrocknet und eingeengt und der verbleibende Rückstand der Chromatographie an Kieselgel mit n-hexan/Essigester = 9 : 1 unterworfen. Man erhält 3,9 g (28%) cis-1-(1,2,4-Triazol-1-yl-1-chlor-methyl)-1-(4-fluorphenyl)-2-(2-fluorphenyl)-cyclopropan als 2 : 1-Diastereomerengemisch (Verbindung Nr. 20).

Entsprechend Beispiel 1 und 2 können die in der Tabelle aufgeführten Verbindungen hergestellt werden.

Tabelle

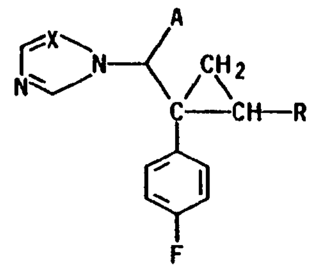

| Bsp.-Nr. | R | A | X | Schmp./IR | Isomer |
|---|---|---|---|---|---|
| 1 | $2-Cl-C_6H_4$ | H | N | 97-101°C | cis |
| 2 | $2-Cl-C_6H_4$ | H | CH | 230°C | cis |
| 3 | $2-Cl-C_6H_4$ | F | N | | |
| 4 | $2-Cl-C_6H_4$ | Cl | N | 144-148°C | Enantiomerengemisch |
| 5 | $2-Cl-C_6H_4$ | Cl | CH | 105-110°C | D1:D2 = 3:1 |
| 6 | $2-Cl-C_6H_4$ | Br | N | | |
| 7 | $2-Cl-C_6H_4$ | Br | CH | | |
| 8 | $3-Cl-C_6H_4$ | H | N | | |
| 9 | $3-Cl-C_6H_4$ | H | CH | | |
| 10 | $3-Cl-C_6H_4$ | Cl | N | | |
| 11 | $4-Cl-C_6H_4$ | H | N | | |
| 12 | $4-Cl-C_6H_4$ | H | CH | | |
| 13 | $4-Cl-C_6H_4$ | Cl | N | | |
| 14 | $4-Cl-C_6H_4$ | Cl | CH | | |
| 15 | $4-Cl-C_6H_4$ | Br | N | | |
| 16 | $4-Cl-C_6H_4$ | Br | CH | | |

Tabelle (Fortsetzung)

| Bsp.-Nr. | R | A | X | Schmp./IR | Isomer |
|---|---|---|---|---|---|
| 17 | 2-F-$C_6H_4$ | H | N | | |
| 18 | 2-F-$C_6H_4$ | H | CH | | |
| 19 | 2-F-$C_6H_4$ | F | N | | |
| 20 | 2-F-$C_6H_4$ | Cl | N | 1513, 1496, 1236, 840, 819, 760 $cm^{-1}$ | D1:D2 = 2:1 |
| 21 | 2-F-$C_6H_4$ | Cl | CH | | |
| 22 | 4-F-$C_6H_4$ | H | N | | |
| 23 | 4-F-$C_6H_4$ | H | CH | | |
| 24 | 4-F-$C_6H_4$ | F | N | | |
| 25 | 4-F-$C_6H_4$ | Cl | N | | |
| 26 | 4-F-$C_6H_4$ | Br | N | | |
| 27 | 2,4-$F_2$-$C_6H_3$ | H | N | | |
| 28 | 2,4-$F_2$-$C_6H_3$ | Cl | N | | |
| 29 | 2,4-$F_2$-$C_6H_3$ | F | N | | |
| 30 | 2-Cl-4-F-$C_6H_3$ | H | N | | |
| 31 | 2-Cl-4-F-$C_6H_3$ | H | CH | | |
| 32 | 2-Cl-4-F-$C_6H_3$ | F | N | | |
| 33 | 2-Cl-4-F-$C_6H_3$ | Cl | N | | |
| 34 | 2-Cl-4-F-$C_6H_3$ | Br | N | | |
| 35 | 2-$CF_3$-$C_6H_4$ | H | N | 1514, 1315, 1162, 1121, 839, 769 $cm^{-1}$ | cis |
| 36 | 2-$CF_3$-$C_6H_4$ | H | CH | 1513, 1315, 1162, 1121, 838, 769 $cm^{-1}$ | cis |

Tabelle (Fortsetzung)

| Bsp.-Nr. | R | A | X | Schmp./IR | Isomer |
|---|---|---|---|---|---|
| 37 | $2-CF_3-C_6H_4$ | F | N | | |
| 38 | $2-CF_3-C_6H_4$ | Cl | N | | |
| 39 | $2-CF_3-C_6H_4$ | Br | N | | |
| 40 | $3-CF_3-C_6H_4$ | H | N | | |
| 41 | $4-CF_3-C_6H_4$ | H | N | | |
| 42 | $4-CF_3-C_6H_4$ | Cl | N | | |
| 43 | $2-OCH_3-C_6H_4$ | H | N | | |
| 44 | $2-OCH_3-C_6H_4$ | H | CH | | |
| 45 | $2-OCH_3-C_6H_4$ | Cl | N | | |
| 46 | $3-OCH_3-C_6H_4$ | H | N | | |
| 47 | $4-OCH_3-C_6H_4$ | H | N | | |
| 48 | $4-OCH_3-C_6H_4$ | H | CH | | |
| 49 | $2,3-Cl_2-C_6H_3$ | H | N | | |
| 50 | $2,5-Cl_2-C_6H_3$ | H | N | | |
| 51 | $2,6-Cl_2-C_6H_3$ | H | N | | |
| 52 | $2-Br-C_6H_4$ | H | N | | |
| 53 | $3-Br-C_6H_4$ | H | N | | |
| 54 | $4-Br-C_6H_4$ | H | N | | |
| 55 | $3-NO_2-C_6H_4$ | H | N | | |
| 56 | $3-NO_2-C_6H_4$ | Cl | N | | |
| 57 | $4-NO_2-C_6H_4$ | H | N | | |
| 58 | $2,4-OCH_3-C_6H_3$ | H | N | | |
| 59 | $2,4-OCH_3-C_6H_3$ | H | N | | |
| 60 | $4-C_2H_5-C_6H_4$ | H | N | | |

Tabelle (Fortsetzung)

| Bsp.-Nr. | R | A | X | Schmp./IR | Isomer |
|---|---|---|---|---|---|
| 61 | 4-O-tert.-butyl-$C_6H_4$ | H | N | | |
| 62 | 4-O-Phenyl-$C_6H_4$ | H | N | | |
| 63 | 3-Pyridyl | H | N | | |
| 64 | Cyclopropyl | H | N | | |
| 65 | Cyclopentyl | H | N | | |
| 66 | Cyclopentyl | H | CH | | |
| 67 | Cyclopentyl | Cl | N | | |
| 68 | Cyclohexyl | H | N | | |
| 69 | Cyclohexyl | H | CH | | |
| 70 | Cyclohexyl | F | N | | |
| 71 | Cyclohexyl | Cl | N | | |
| 72 | Cyclohexyl | Cl | CH | | |
| 73 | Cyclohexyl | Br | N | | |
| 74 | 3-Cyclohexenyl | H | N | | |
| 75 | Norbornyl | H | N | | |
| 76 | 4-Tetrahydropyranyl | H | N | | |
| 77 | $C_6H_5$ | H | N | | |
| 78 | $C_6H_5$ | H | CH | | |
| 79 | $C_6H_5$ | Cl | N | | |
| 80 | $C_6H_5$ | Cl | CH | | |
| 81 | $C_6H_5$ | F | N | | |
| 82 | $CH_3$ | H | N | | |
| 83 | $C_2H_5$ | H | N | | |

Tabelle (Fortsetzung)

| Bsp.-Nr. | R | A | X | Schmp./IR | Isomer |
|---|---|---|---|---|---|
| 84 | tert.-$C_4H_9$ | H | N | | |
| 85 | tert.-$C_4H_9$ | H | CH | | |
| 86 | tert.-$C_4H_9$ | Cl | N | | |
| 87 | $C_{10}H_7$ | H | N | | |
| 88 | $C_{10}H_7$ | H | CH | | |
| 89 | $C_{12}H_9$ | H | N | | |
| 90 | $C_{12}H_9$ | H | CH | | |

D1:D2 = Mengenverhältnis der hergestellten Diastereomeren

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse – wie Gurken, Bohnen und Kürbisgewächse –.

Die neuen Verbindungen sind insbesondere geeignet zur Bekämpfung folgender Pflanzenkrankheiten :

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosphorella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmorpara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Verbindungen können praktisch alle Entwicklungstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,
b) von dem Zeitpunkt der Applikation, bezogen auf das Entwicklungsstadium der Pflanze und von der Jahreszeit,
c) von dem Applikationsort und -verfahren (Samenbeize, Bodenbehandlung oder Blattapplikation)
d) von klimatischen faktoren, z.B. Temperatur, Niederschlagsmenge, außerdem auch Tageslänge und Lichtintensität
e) von der Bodenbeschaffenheit (einschließlich Düngung),
f) von der Formulierung bzw. Anwendungsform des Wirkstoffs und schließlich
g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, werden einige nachstehend erwähnt.

A.

Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf ; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Reis, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und durch besonders frostanfälligen) Blatt- bzw. Pflanzenmasse gehemmt.

Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und – wegen der relativ geringen Blatt- und Pflanzenmasse – dem Befall mit verschiedenen Krankheiten (z.B. Pilzkrankheit) vorgebeugt werden. Die Hemmung des vegetativen Wachstums ermöglicht außerdem bei vielen Kulturpflanzen die dichtere Bepflanzung des Bodens, so daß ein Mehrertrag, bezogen aus die Bodenfläche, erzielt werden kann.

B.

Mit den neuen Mitteln lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.

C.

Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftichem Interesse ist beispielsweise die Ernteerleicherung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wesentlich.

D.

Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite des Stomata reduziert wird
- eine dickere Epidermis und Cuticula ausgebildet werden
- die Durchwurzelung des Bodens verbessert wird
- das Mikroklima im Pflanzenbestand durch einen kompakteren Wuchs günstig beeinflußt wird.

Die erfindungsgemäß zu verwendeten Wirkstoffe können den Kulturpflanzen sowohl von Samen her (als Saatgutbeizmittel) als auch über den Boden, d.h. durch die Wurzel sowie über das Blatt zugeführt werden.

Infolge der hohen Pflanzenverträglichkeit kann die Aufwandmenge als Wachstumsregulatoren stark variiert werden.

Bei der Saatgutbehandlung werden im allgemeinen Wirkstoffmengen von 0,001 bis 50 g, vorzugsweise 0,01 bis 10 g je Kilogramm Saatgut benötigt.

Für die Blatt- und Bodenbehandlung sind im allgemeinen Gaben von 0,01 bis 10 kg/ha, bevorzugt 0,02 bis 3 kg/ha, als ausreichend zu betrachten.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage : Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), amine (z.B. Ethanolamin, Dimethylformamid) und Wasser ; Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen als Fungizide liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind :

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 35 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teil Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 2 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mil Ricinusöl besteht. Durch Eingießen und feines Verteilen der Lösung in Wasser erhält man eine wäßrige Dispersion

IV. 20 Gew.-Teile der Verbindung Nr. 36 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 35 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 36 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid ;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat ;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,

2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3--chlor-2-aminopyridin,
1-((bis-(4-Fluorphenyl)-methylsilyl)-methyl)-1H-1,2,4-triazol.

Anwendungsbeispiele

Als Vergleichswirkstoffe wurden die Verbindungen 1-(1,2,4-Triazol-1-yl-methyl)-1-(4-chlorphenyl)-2-(2-chlorphenyl)-cyclopropan (A) und 1-(1,2,4-Triazol-1-ylmethyl)-1-(4-fluorphenyl)-2-(2,4-dichlorphenyl)-cyclopropan (B) – bekannt aus EP 121 081 – benutzt.

Anwendungsbeispiel 1

Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95%) gestellt. Während dieser Zeit keimten die Sporen aus und die Keimschläuche drangen in das Blattgewebe ein. Die infizierten Pflanzen wurden anschließend mit wäßrigen Spritzbrühen, die 80% (Gew.%) Wirkstoff und 20% Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70% relativer Luftfeuchte aufgestellt. Nach 8 Tagen wurde das Ausmaß der Rostpilzentwicklung auf den Blättern ermittelt.

Das Ergebnis zeigt, daß die Wirkstoffe 1 und 35 bei der Anwendung als 0,0015%ige (Gew.%) Spritzbrühen eine bessere fungizide Wirkung zeigen (100%) als die bekannten Vergleichswirkstoffe A und B (90%).

Anwendungsbeispiel 2

Wirksamkeit gegen Rebenperonospora (Plasmopara viticola)

Blätter von Topfreben der Sorte "Müller Thurgau" wurden mit wäßrigen Spritzbrühen besprüht. Um die Wirkungsdauer der Wirkstoffe beurteilen zu können, wurden die Pflanzen nach dem Antrocknen der Spritzbeläge 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, daß die Wirkstoffe 2 und 36 bei der Anwendung als 0,0125%ige Spritzbrühe eine bessere fungizide Wirkung zeigen (90%) als der bekannte Vergleichswirkstoff B (20%).

Anwendungsbeispiel 3

Wirksamkeit gegen Helminthosporium teres (Pyrenophora teres)

Gerstenkeimlinge der Sorte "Igri" wurden im Zweiblattstadium mit wäßrigen Suspensionen, die 80% Wirkstoff und 20% Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in einer Klimakammer mit hoher Luftfeuchtigkeit bei 18°C gestellt. Anschließend wurden die Pflanzen im Gewächshaus bei 20 bis 22°C und 70% relativer Luftfeuchtigkeit für weitere 5 Tage kultiviert. Dann wurde das Ausmaß der Symptomentwicklung ermittelt.

Das Ergebnis zeigt, daß die Wirkstoffe 1, 35 und 36 bei der Anwendung als 0,0125%ige Spritzbrühe eine sehr gute fungizide Wirkung zeigen (100%).

Zur Bestimmung der wachstumsregulierenden Eigenschaften der Prüfsubstanzen wurden Testpflanzen auf ausreichend mit Nährstoffen versorgtem Kultursubstrat in Kunststoffgefäßen (c. 12,5 cm Durchmesser) angezogen.

Im Vorauflaufverfahren wurden die Testsubstanzen in wäßriger Aufbereitung am Tage der Einsaat auf das Saatbett gegossen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz diente der bekannte Wirkstoff Chlorcholinchlorid (C).

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen zu entnehmen :

Vergleichssubstanz :

C

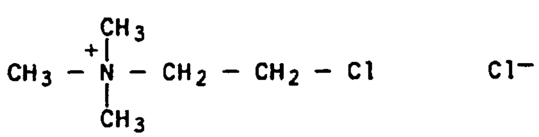

Anwendungsbeispiel 4

Sommergerste, Sorte "Aramir"
Vorauflauf-Bodenbehandlung

| Wirkstoff-Nr. | Konzentration mg Wirkstoff/Gefäß | Wuchshöhen relativ |
|---|---|---|
| unbehandelt | - | 100 |
| C | 6 | 100 |
| 1 | 6 | 67,8 |
| 35 | 6 | 60,2 |

Anwendungsbeispiel 5

Sonnenblumen, Sorte "Sorex"
Nachauflauf-Bodenbehandlung

| Wirkstoff-Nr. | Konzentration mg Wirkstoff/Gefäß | Wuchshöhen relativ |
|---|---|---|
| unbehandelt | - | 100 |
| C | 6 | 100 |
| 35 | 6 | 90,3 |

Anwendungsbeispiel 6

Rasen
Blattbehandlung

| Wirkstoff-Nr. | Konzentration mg Wirkstoff/Gefäß | Wuchshöhen relativ |
|---|---|---|
| unbehandelt | - | 100 |
| C | 1,5 | 100,8 |
| | 6 | 94,5 |
| 1 | 1,5 | 75,6 |
| | 6 | 25,2 |
| 35 | 1,5 | 88,2 |
| | 6 | 25,2 |

## Ansprüche

1. Azolylmethylcyclopropane der Formel

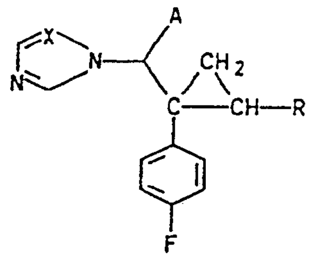

I

in welcher

R $C_1$-$C_8$-Alkyl, $C_3$-$C_3$-Cycloalkyl, $C_5$-$C_8$-Cycloalkenyl, Tetrahydropyranyl, Norbornyl, Pyridyl, Naphthyl, Biphenyl oder Phenyl bedeutet, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,

A den Rest H, F, Cl oder Br bedeutet,

X den Rest CH oder N bedeutet,

sowie deren für Pflanzen verträgliche Säureadditionssalze und Metallkomplexe, außer den Verbindungen, in denen gleichzeitig A Wasserstoff und R 2,4-Dichlorphenyl bedeuten.

2. Azolylmethylcyclopropane der Formel I gemäß Anspruch 1, in denen R die Phenylgruppe bedeutet, welche unsubstituiert ist oder durch einen oder zwei Substituenten substituiert ist, die Fluor, Chlor, Brom oder die Trifluormethylgruppe bedeuten.

3. Verfahren zu Herstellung der Azolylmethylcyclopropane der Formel I

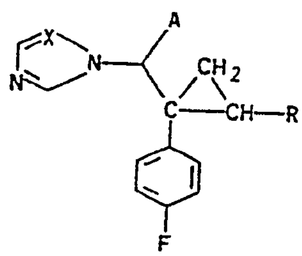

I

in welcher

R $C_1$-$C_8$-Alkyl, $C_3$-$C_8$-Cycloalkyl, $C_5$-$C_8$-Cycloalkenyl, Tetrahydropyranyl, Norbornyl, Pyridyl, Naphthyl, Biphenyl oder Phenyl bedeutet, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,

A den Rest H

X den Rest CH oder N bedeutet,

sowie deren für Pflanzen verträgliche Säureadditionssalze und Metallkomplexe, außer den Verbindungen, in denen gleichzeitig A Wasserstoff und R 2,4-Dichlorphenyl bedeuten, dadurch gekennzeichnet, daß man eine Verbindung der Formel II

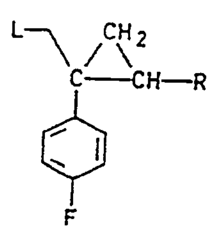

II,

in welcher R die oben angegebene Bedeutung hat und L eine nukleophil substituierbare Abgangsgruppe darstellt, mit einer Verbindung der Formel III

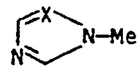

III,

in der Me ein Wasserstoffatom, ein Metallatom oder eine Trimethylsilylgruppe bedeutet und X die oben angegebene Bedeutung hat, umsetzt und die so erhaltenen Verbindungen gegebenenfalls in ihre Salze mit für Pflanzen verträglichen Säuren oder Metallkomplexe überführt.

4. Verfahren zur Herstellung der Azolylmethylcyclopropane der Formel I

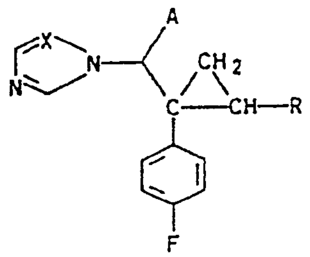

I

in welcher

R $C_1$-$C_8$-Alkyl, $C_3$-$C_8$-Cycloalkyl, $C_5$-$C_8$-Cycloalkenyl, Tetrahydropyranyl, Norbornyl, Pyridyl, Naphthyl, Biphenyl oder Phenyl bedeutet, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,

A den Rest F, Cl oder Br bedeutet,

X den Rest CH oder N bedeutet,

sowie deren für Pflanzen verträgliche Säureadditionssalze und Metallkomplexe, dadurch gekennzeichnet, daß man eine Verbindung der Formel V

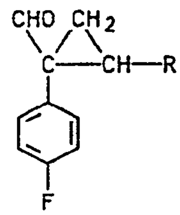

V

in welcher R die oben angegebene Bedeutung hat, mit einer Verbindung der Formel III

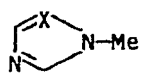

III

in der Me ein Wasserstoffatom, ein Metallatom oder eine Trimethylsilylgruppe bedeutet und X die oben angegebene Bedeutung hat, in Gegenwart des entsprechenden Thionylhalogenids zur Umsetzung bringt.

5. Fungizides Mittel, enthaltend einen Trägerstoff und ein Azolylmethylcyclopropan der Formel I

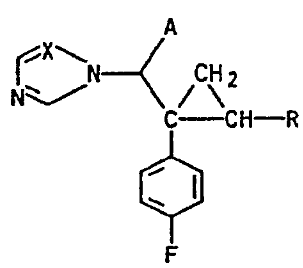

I

in welcher

R $C_1$-$C_8$-Alkyl, $C_3$-$C_8$-Cycloalkyl, $C_5$-$C_8$-Cycloalkenyl, Tetrahydropyranyl, Norbornyl, Pyridyl, Naphthyl, Biphenyl oder Phenyl bedeutet, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,

A den Rest H, F, Cl oder Br bedeutet,

X den Rest CH oder N bedeutet,

oder dessen für Pflanzen verträgliches Säureadditionssalz oder Metallkomplex, außer den Verbindungen, in denen gleichzeitig A Wasserstoff und R 2,4-Dichlorphenyl bedeuten.

6. Verfahren zur Bekämpfung von Pilzen, dadurch gekennzeichnet, daß man eine fungizid wirksame Menge eines Azolylmethylcyclopropans der Formel I

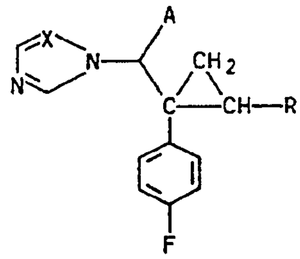

I

in welcher

R $C_1$-$C_8$-Alkyl, $C_3$-$C_8$-Cycloalkyl, $C_5$-$C_8$-Cycloalkenyl, Tetrahydropyranyl, Norbornyl, Pyridyl, Naphthyl, Biphenyl oder Phenyl bedeutet, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,

A den Rest H, F, Cl oder Br bedeutet,

X den Rest CH oder N bedeutet,

oder dessen für Pflanzen verträgliche Säureadditionssalze oder Metallkomplexe, außer den Verbindungen, in denen gleichzeitig A Wasserstoff und R 2,4-Dichlorphenyl bedeuten, auf die Pilze oder durch Pilzbefall bedrohte Materialien, Flächen, Pflanzen oder Saatgüter einwirken läßt

7. Azolylmethylcyclopropan der Formel I gemäß Anspruch 1, wobei R 2-Chlorphenyl, A Wasserstoff und X N bedeutet.

8. Azolylmethylcyclopropan der Formel I gemäß Anspruch 1, wobei R 2-Trifluormethylphenyl, A Wasserstoff und X N bedeutet.

9. Wachstumsregulierendes Mittel, enthaltend einen Trägerstoff und eine wachstumsregulierende Menge eines Azolylmethylcyclopropans der Formel

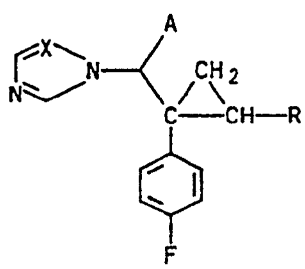

I

in welcher

R $C_1$-$C_8$-Alkyl, $C_3$-$C_8$-Cycloalkyl, $C_5$-$C_8$-Cycloalkenyl, Tetrahydropyranyl, Norbornyl, Pyridyl, Naphthyl, Biphenyl oder Phenyl bedeutet,wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,

A den Rest H, F, Cl oder Br bedeutet,

X den Rest CH oder N bedeutet,

oder dessen für Pflanzen verträglichem Säureadditionssalz oder Metallkomplex, außer der Verbindungen, in denen gleichzeitig A Wasserstoff und R 2,4-Dichlorphenyl bedeuten.

10. Verfahren zur Regulierung des Pflanzenwachstums, dadurch gekennzeichnet, daß man die Pflanzen, den Boden oder die Saatgüter behandelt mit einer wachstumsregulierenden Menge eines Azolylmethylcyclopropans der Formel

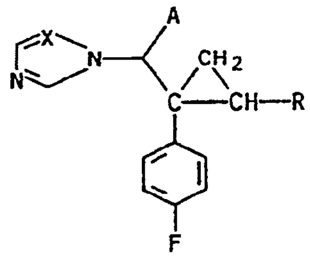

I

in welcher

R $C_1$-$C_8$-Alkyl, $C_3$-$C_8$-Cycloalkyl, $C_5$-$C_8$-Cycloalkenyl, Tetrahydropyranyl, Norbornyl, Pyridyl, Naphthyl, Biphenyl oder Phenyl bedeutet, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,

A den Rest H, F, Cl oder Br bedeutet,

X den Rest CH oder N bedeutet,

oder dessen für Pflanzen verträglichem Säureadditionssalz oder Metallkomplex, außer den Verbindungen, in denen gleichzeitig A Wasserstoff und R 2,4-Dichlorphenyl bedeuten.

## Claims

1. An azolylmethylcyclopropane of the formula

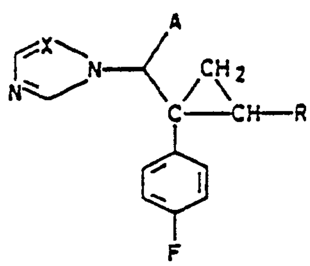

I

where R is $C_1$-$C_8$-alkyl, $C_3$-$C_8$-cycloalkyl, $C_5$-$C_8$-cycloalkenyl, tetrahydropyranyl, norbornyl, pyridyl, naphthyl, biphenyl or phenyl, and these radicals may be monosubstituted to trisubstituted by halogen, nitro, phenoxy, amino, alkyl, alkoxy or haloalkyl, each of 1 to 4 carbon atoms, A is H, F, Cl or Br, and X is CH or N, and its plant-tolerated acid addition salts and metal complexes with the exception of compounds in which both A is hydrogen and R is 2,4-dichlorophenyl.

2. An azolylmethylcyclopropane of the formula I as claimed in claim 1, where R is phenyl which is unsubstituted or monosubstituted or disubstituted by fluorine, chlorine, bromine or trifluoromethyl.

3. A process for the manufacture of azolylmethylcyclopropanes of the formula I

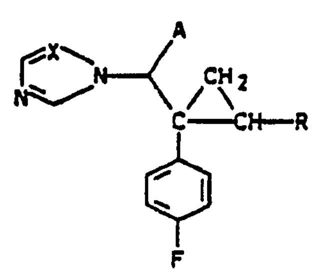

where R is $C_1$-$C_8$-alkyl, $C_3$-$C_8$-cycloalkyl, $C_5$-$C_8$-cycloalkenyl, tetrahydropyranyl, norbornyl, pyridyl, naphthyl, biphenyl or phenyl, and these radicals may be monosubstituted to trisubstituted by halogen, nitro, phenoxy, amino, alkyl, alkoxy or haloalkyl, each of 1 to 4 carbon atoms, A is H, X is CH or N, and their plant-tolerated acid addition salts and metal complexes, with the exception of compounds in which R is 2,4-dichlorophenyl, wherein a compound of the formula II

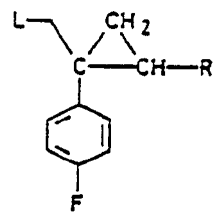

II,

where R has the above meanings and L is a nucleophilically substituted leaving group, is reacted with a compound of the formula III

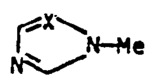

III,

where Me is a hydrogen atom, a metal atom or a trimethylsilyl group and X has the above meanings, and the compound thus obtained is if desired converted into a salt with plant-tolerated acids, or a metal complex.

4. A process for the manufacture of azolylmethylcyclopropanes of the formula I

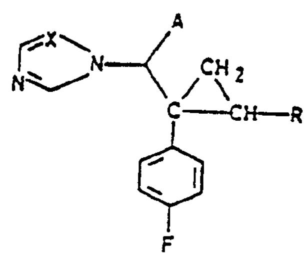

I

where R is $C_1$-$C_8$-alkyl, $C_3$-$C_8$-cycloalkyl, $C_5$-$C_8$-cycloalkenyl, tetrahydropyranyl, norbornyl, pyridyl, naphthyl, biphenyl or phenyl, and these radicals may be monosubstituted to trisubstituted by halogen, nitro, phenoxy, amino, alkyl, alkoxy or haloalkyl, each of 1 to 4 carbon atoms, A is F, Cl or Br, and their plant-tolerated acid addition salts and metal complexes, wherein a compound of the formula V

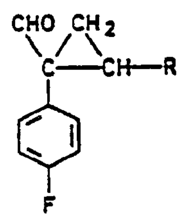

V

where R has the above meanings, is reacted with a compound of the formula III

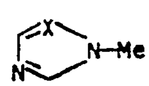

III

where Me is a hydrogen atom, a metal atom or a trimethylsilyl group and X has the above meanings, in the presence of the corresponding thionyl halide.

5. A fungicidal agent containing a carrier and an azolylmethylcyclopropane of the formula I

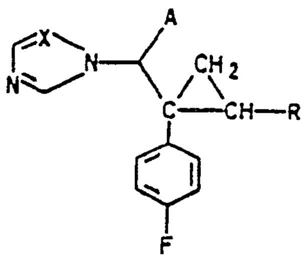

I

where R is $C_1$-$C_8$-alkyl, $C_3$-$C_8$-cycloalkyl, $C_5$-$C_8$-cycloalkenyl, tetrahydropyranyl, norbornyl, pyridyl, naphthyl, biphenyl or phenyl, and these radicals may be monosubstituted to trisubstituted by halogen, nitro, phenoxy, amino, alkyl, alkoxy or haloalkyl, each of 1 to 4 carbon atoms, A is H, F, Cl or Br, and X is CH or N, and its plant-tolerated acid addition salts and metal complexes with the exception of compounds in which both A is hydrogen and R is 2,4-dichlorophenyl.

6. A process for combating fungi, wherein a fungicidally effective amount of an azolylmethylcyclopropane of the formula I

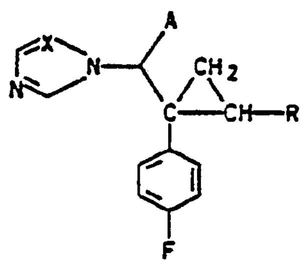

I

where R is $C_1$-$C_8$-alkyl, $C_3$-$C_8$-cycloalkyl, $C_5$-$C_8$-cycloalkenyl, tetrahydropyranyl, norbornyl, pyridyl, naphthyl, biphenyl or phenyl, and these radicals may be monosubstituted to trisubstituted by halogen, nitro, phenoxy, amino, alkyl, alkoxy or haloalkyl, each of 1 to 4 carbon atoms, A is H, F, Cl or Br, and X is CH or N, and its plant-tolerated acid addition salts and metal complexes with the exception of compounds in which both A is hydrogen and R is 2,4-dichlorophenyl, is allowed to act on the fungi, or on materials, areas, plants or seed threatened by fungus attack.

7. An azolylmethylcyclopropane of the formula I as claimed in claim 1, where R is 2-chlorophenyl, A is hydrogen and X is N.

8. An azolylmethylcyclopropane of the formula I as claimed in claim 1, where R is 2-trifluoromethylphenyl, A is hydrogen and X is N.

9. A growth-regulating agent containing a carrier and a growth-regulating amount of an azolylmethylcyclopropane of the formula

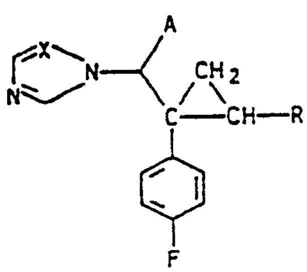

I

where R is $C_1$-$C_8$-alkyl, $C_3$-$C_8$-cycloalkyl, $C_5$-$C_8$-cycloalkenyl, tetrahydropyranyl, norbornyl, pyridyl, naphthyl, biphenyl or phenyl, and these radicals may be monosubstituted to trisubstituted by halogen, nitro, phenoxy, amino, alkyl, alkoxy or haloalkyl, each of 1 to 4 carbon atoms, A is H, F, Cl or Br, and X is CH or N, and its plant-tolerated acid addition salts and metal complexes with the exception of compounds in which both A is hydrogen and R is 2,4-dichlorophenyl.

10. A process for regulating plant growth, wherein the plants, the soil or the seed are treated with a growth-regulating amount of an azolylmethylcyclopropane of the formula

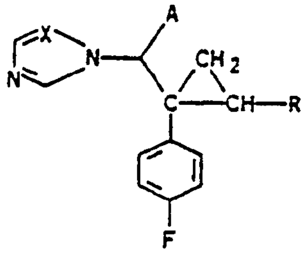

I

where R is $C_1$-$C_8$-alkyl, $C_3$-$C_8$-cycloalkyl, $C_5$-$C_8$-cycloalkenyl, tetrahydropyranyl, norbornyl, pyridyl, naphthyl, biphenyl or phenyl, and these radicals may be monosubstituted to trisubstituted by halogen, nitro, phenoxy, amino, alkyl, alkoxy or haloalkyl, each of 1 to 4 carbon atoms, A is H, F, Cl or Br, and X is CH or N, and its plant-tolerated acid addition salts and metal complexes with the exception of compounds in which both A is hydrogen and R is 2,4-dichlorophenyl.

**Revendications**

1. Azolylméthylcyclopropanes de formule

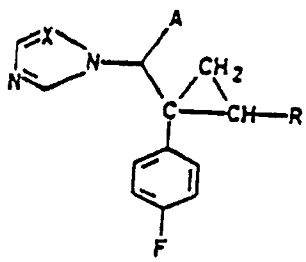

I

dans laquelle

R représente un groupe alkyle en C1-C8, cycloalkyle en C3-C8, cycloalcényle en C5-C8, tétrahydropyrannyle, norbornyle, pyridyle, naphtyle, biphényle ou phényle, ces radicaux pouvant porter un à trois substituants choisis parmi les halogènes, les groupes nitro, phénoxy, amino, alkyle, alcoxy ou halogénoalkyle contenant chacun 1 à 4 atomes de carbone,

A représente H, F, Cl ou Br,

X représente CH ou N,

et leurs sels formés par addition avec des acides et complexes métalliques tolérés par les végétaux, à l'exception des composés dans lesquels, simultanément, A représente l'hydrogène et R le groupe 2,4-dichlorophényle.

2. Azolylméthylcyclopropanes de formule I selon la revendication 1, dans laquelle R représente le groupe phényle non substitué ou portant un ou deux substituants choisis parmi le fluor, le chlore, le brome ou les groupes trifluorométhyle.

3. Procédé de préparation des azolylméthylcyclopropanes de formule I

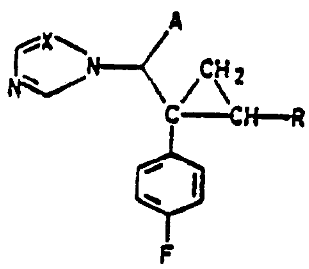

I

dans laquelle

R représente un groupe alkyle en C1-C8, cycloalkyle en C3-C8, cycloalcényle en C5-C8, tétrahydropyrannyle, norbornyle, pyridyle, naphtyle, biphényle ou phényle, tous ces radicaux pouvant porter un à trois substituants choisis parmi les halogènes, les groupes nitro, phénoxy, amino, alkyle, alcoxy ou halogénoalkyle contenant chacun 1 à 4 atomes de carbone,

A représente H,

X représente CH ou N,

et de leurs sels formés par addition avec des acides et complexes métalliques tolérés par les végétaux, à l'exception des composés dans lesquels, simultanément, A représente l'hydrogène et R le groupe 2,4-dichlorophényle,

caractérisé en ce que :

on fait réagir un composé de formule II

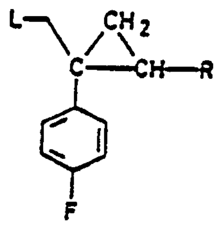

II,

dans laquelle R a les significations indiquées ci-dessus et L représente un groupe éliminable apte à une substitution nucléophile, avec un composé de formule III

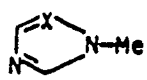

III,

dans laquelle Me représente un atome d'hydrogène, un atome métallique ou un groupe triméthylsilyle et X a les significations indiquées ci-dessus, et le cas échéant on convertit les composés ainsi obtenus en leurs sels d'acides ou complexes métalliques tolérés par les végétaux.

4. Procédé de préparation des azolylméthylcyclopropanes de formule I

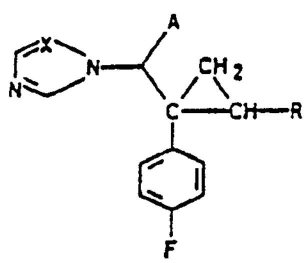

I

dans laquelle

R représente un groupe alkyle en C1-C8, cycloalkyle en C3-C8, cycloalcényle en C5-C8, tétrahydropyrannyle, norbornyle, pyridyle, naphtyle, biphényle ou phényle, tous ces radicaux pouvant porter un à trois substituants choisis parmi les halogènes, les groupes nitro, phénoxy, amino, alkyle, alcoxy ou halogénoalkyle contenant chacun 1 à 4 atomes de carbone,

A représente F, Cl ou Br,

X représente CH ou N

et de leurs sels formés par addition avec des acides et complexes métalliques tolérés par les végétaux, caractérisé en ce que l'on fait réagir un composé de formule V

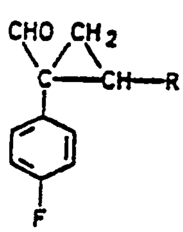

V

dans laquelle R a les significations indiquées ci-dessus, avec un composé de formule III

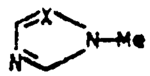

III

dans laquelle Me représente un atome d'hydrogène, un atome métallique ou un groupe triméthylsilyle et X a les significations indiquées ci-dessus, en présence de l'halogénure de thionyle correspondant.

5. Produit fongicide contenant un véhicule et un azolylméthylcyclopropane de formule I

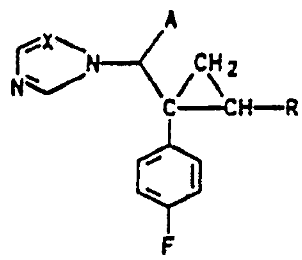

I

dans laquelle

R représente un groupe alkyle en C1-C8, cycloalkyle en C3-C8, cycloalcényle en C5-C8, tétrahydropyrannyle, norbornyle, pyridyle, naphtyle, biphényle ou phényle, tous ces radicaux pouvant porter un à trois substituants choisis parmi les halogènes, les groupes nitro, phénoxy, amino, alkyle, alcoxy ou halogénoalkyle contenant chacun 1 à 4 atomes de carbone,

A représente H, F, Cl ou Br,

X représente CH ou N,

ou l'un de leurs sels formés par addition avec un acide ou l'un de leurs complexes métalliques, tolérés par les végétaux, à l'exception des composés dans lesquels, simultanément, A représente l'hydrogène et R le groupe 2,4-dichlorophényle.

6. Procédé pour combattre les mycètes, caractérisé en ce que l'on fait agir sur les mycètes ou sur les matières, surfaces, végétaux ou semences menacés par une attaque de mycètes, une quantité fongicide efficace d'un azolylméthylcyclopropane de formule I

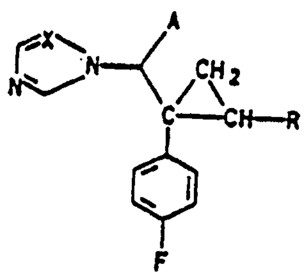

I

dans laquelle

R représente un groupe alkyle en C1-C8, cycloalkyle en C3-C8, cycloalcényle en C5-C8, tétrahydropyrannyle, norbornyle, pyridyle, naphtyle, biphényle ou phényle, tous ces radicaux pouvant porter un à trois substituants choisis parmi les halogènes, les groupes nitro, phénoxy, amino, alkyle, alcoxy ou halogénoalkyle contenant chacun 1 à 4 atomes de carbone,

A représente H, F, Cl ou Br,

X représente CH ou N,

où de l'un de ses sels formés par addition avec des acides ou complexes métalliques tolérés par les végétaux, à l'exception des composés dans lesquels, simultanément, A représente l'hydrogène et R représente le groupe 2, 4-dichlorophényle.

7. Azolylméthylcyclopropane de formule I selon la revendication 1, dans laquelle R représente le groupe 2-chlorophényle, A représente l'hydrogène et X représente N.

8. Azolylméthylcyclopropane de formule I selon la revendication 1, dans laquelle R représente le groupe

2-trifluorométhylphényle, A l'hydrogène et X représente N.

9. Produit régulateur de croissance, contenant un véhicule et une quantité efficace pour la régulation de croissance, d'un azolylméthylcyclopropane de formule

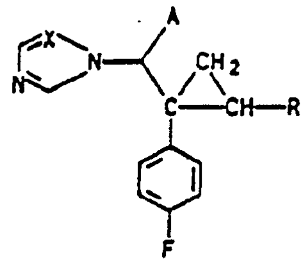

I

dans laquelle

R représente un groupe alkyle en C1-C8, cycloalkyle en C3-C8, cycloalcényle en C5-C8, tétrahydropyrannyle, norbornyle, pyridyle, naphtyle, biphényle ou phényle, tous ces radicaux pouvant porter un à trois substituants choisis parmi les halogènes, les groupes nitro, phénoxy, amino, alkyle, alcoxy ou halogénoalkyle contenant chacun 1 à 4 atomes de carbone,

A représente H, F, Cl ou Br,

X représente CH ou N,

ou de l'un de ses sels formés par addition les acides ou complexes métalliques tolérés par les végétaux, à l'exception des composés dans lesquels, simultanément, A représente l'hydrogène et R le groupe 2,4-dichlorophényle.

10. Procédé pour la régulation de la croissance des végétaux, caractérisé en ce que l'on traite les végétaux, le sol ou les semences par une quantité efficace pour la régulation de croissance d'un azolylméthylcyclopropane de formule

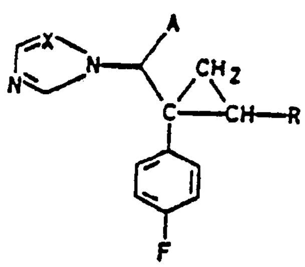

I

dans laquelle

R représente un groupe alkyle en C1-C8, cycloalkyle en C3-C8, cycloalcényle en C5-C8, tétrahydropyrannyle, norbornyle, pyridyle, naphtyle, biphényle ou phényle, tous ces radicaux pouvant porter un à trois substituants choisis parmi les halogènes, les groupes nitro, phénoxy, amino, alkyle, alcoxy ou halogénoalkyle contenant chacun 1 à 4 atomes de carbone,

A représente H, F, Cl ou Br,

X représente CH ou N,

ou de l'un de ses sels formés par addition avec des acides ou complexes métalliques tolérés par les végétaux, à l'exception des composés dans lesquels, simultanément, A représente l'hydrogène et R le groupe 2,4-dichlorophényle.